# EUROPÄISCHE PATENTSCHRIFT

(11) **EP 0 755 954 B1**
(45) Veröffentlichungstag und Bekanntmachung des Hinweises auf die Patenterteilung: **06.05.1999**
(21) Anmeldenummer: 96111157.2
(22) Anmeldetag: 11.07.1996
(51) Int. Cl.: C08G 18/02, C08G 18/79, C08G 18/18

(54) **Verfahren zur Herstellung von Isocyanuratgruppen enthaltenden Polyisocyanaten aus aliphatischen und aromatischen Isocyanatverbindungen**
Process for the preparation of isocyanurate group containing polyisocyanates from aliphatic and aromatic isocyanate compounds
Procédé pour la préparation de polyisocyanates contenant des groupes d'isocyanurate à partir de composants d'isocyanates aliphatiques et aromatiques

(30) Priorität: 24.07.1995 DE 19526920
(43) Veröffentlichungstag der Anmeldung: 29.01.1997
(73) Patentinhaber: BAYER AG, 51368 Leverkusen (DE)
(72) Erfinder: Brahm, Martin, Dr., 51373 Leverkusen (DE); Dieris, Carl-Gerd, Dr., 41539 Dormagen (DE); Schmalstieg, Lutz, Dr., 50676 Köln (DE); Halpaap, Reinhard, Dr., 51519 Odenthal (DE)

(56) Entgegenhaltungen:
- EP-A- 0 115 298
- EP-A- 0 187 105
- DE-A- 2 616 415
- FR-A- 2 023 423

## Beschreibung

Die Erfindung betrifft ein Verfahren zur Herstellung Isocyanuratgruppen enthaltender Polyisocyanate durch katalytische Trimerisierung einer Mischung aus niedermolekularen Isocyanatkomponenten mit aliphatisch und aromatisch gebundenen Isocyanatgruppen durch Aminosilylverbindungen sowie ihre Verwendung in Beschichtungsmassen.

Die katalytische Trimerisierung von Isocyanatgruppen gehört seit langem zu einer bewährten Modifizierungsreaktion von Diisocyanaten, um Beschichtungsmittel mit hervorragendem Eigenschaftsniveau herzustellen. Man unterscheidet hierbei Trimerisate auf Basis aliphatischer und aromatischer Isocyanatkomponenten.

Auf aliphatischen Diisocyanaten basierende Lackpolyisocyanate weisen in Beschichtungen eine ausgezeichnete Lichtstabilität und Chemikalienbeständigkeit auf und werden beispielsweise in der Automobillackierung eingesetzt. Derartige Polyisocyanate auf Isocyanuratbasis sind bekannt (siehe z.B. J. prakt. Chem. 336, 185-200 (1994)). Als Katalysatoren zur Trimerisierung von aliphatischen Diisocyanaten kommen vorzugsweise Alkyl(aryl)ammoniumhydroxide, Alkoholate, Amine und Aminosilylverbindungen zum Einsatz.

Im Gegensatz hierzu sind Lackpolyisocyanate, hergestellt aus aromatischen Diisocyanaten, hochreaktiv und finden so als schnelltrocknende und hartmachende Vernetzer Verwendung. Als Trimerisationskatalysatoren kommen hierfür vornehmlich Mannichbasen in Frage (z.B. DE-A 2 551 534).

Mischtrimerisate auf Basis aliphatischer und aromatischer Diisocyanate vereinigen synergetisch das Eigenschaftsbild rein aliphatischer bzw. rein aromatischer Analoga und werden bevorzugt eingesetzt, wo neben einer schnellen Aushärtung relativ lichtechte und harte Beschichtungen gefragt sind.

Die Mischtrimerisation von hochreaktiven aromatischen Diisocyanaten mit wenigreaktiven aliphatischen Diisocyanaten ist jedoch schwierig durchführbar.

Lediglich Alkylphosphane, wie Tributylphosphan, sind als geeignete Katalysatoren zur Mischtrimerisation beschrieben (DE-A 1 954 093). Von Nachteil ist hier jedoch die schwierige Handhabung der z. T. selbstentzündlichen Phosphane und die Desaktivierung des Katalysators.

Ein weiteres Verfahren zur Herstellung von Mischtrimerisaten ist aus der DE-A 3 144 672 bekannt. Hier kann eine Mischtrimerisation mit Alkalisalzen nur über ein langsames Zudosieren des hochreaktiven Aromaten zu einer Aliphaten/Katalysatormischung erreicht werden. Die Folge ist eine unkontrollierte Reaktion von aromatischen und aliphatischen Diisocyanaten nebeneinander und keine echte Mischtrimerisation.

Der Einsatz von Aminosilylverbindungen zur Herstellung von Lackpolyisocyanaten ist bekannt, z.B. aus US-A 4 412 073, 4 537 961, 4 675 401, 4 697 014, EP-A 57 653, 89 297 und 187 105. Es wird die katalytische Trimerisation aliphatischer oder aromatischer Diisocyanate beschrieben, nicht jedoch die Trimerisierung von Mischungen aus aliphatischen und aromatischen Isocyanaten.

Gesucht wird deshalb ein Verfahren zur Herstellung Isocyanuratgruppen enthaltender Polyisocyanate mit geringem Monomergehalt bestehend sowohl aus aromatischen als auch aliphatischen Isocyanatverbindungen.

Es wurde nun gefunden, daß ein Gemisch aus aliphatischen und aromatischen Isocyanaten in Gegenwart von Aminosilylverbindungen als Katalysatoren zu Isocyanuraten umgesetzt wird.

Als besonders überraschend muß bei der vorliegenden Erfindung gelten, daß die weniger reaktiven aliphatischen Isocyanate bevorzugt in das Isocyanurat eingebaut werden. Dies läßt sich durch Bestimmung des Restgehaltes an Monomeren direkt nach der Trimerisierung bestätigen.

Gegenstand der Erfindung ist daher ein Verfahren zur Herstellung von Isocyanuratgruppen enthaltenden Polyisocyanaten dadurch gekennzeichnet, daß
10 bis 90 Teile einer niedermolekularen Isocyanatkomponente A) mit aliphatisch gebundenen Isocyanatgruppen, eines mittleren Molekulargewichts von 128 bis 800 und einer mittleren NCO-Funktionalität von 1,7 bis 2,2
   und
90 bis 10 Teile einer niedermolekularen Isocyanatkomponente B) mit aromatisch gebundenen Isocyanatgruppen, eines mittleren Molekulargewichts von 148 bis 800 und einer mittleren NCO-Funktionalität von 1,7 bis 2,2 unter katalytischer Trimerisation
   mit
0,1 bis 10 Teilen einer Aminosilylverbindung C) pro 100 Teile Isocyanatkomponente A) und B) zur Reaktion gebracht werden
und im Anschluß überschüssige destillierbare Isocyanatkomponenten A) und B) bis auf einen Gehalt von unter 0,7 % entfernt werden.

Gegenstand der Erfindung sind auch so hergestellte Produkte und deren Verwendung zur Herstellung von Beschichtungen.

Beim erfindungsgemäßen Verfahren kann eine beliebige Isocyanatkomponente A) mit aliphatischen und/oder cycloaliphatischen Isocyanatgruppen, einem mittleren Molekulargewicht von 128 bis 800, vorzugsweise 128 bis 300 und einer NCO-Funktionalität von 1,7 bis 2,3 vorzugsweise 1,9 bis 2,1 und besonders bevorzugt 2 eingesetzt werden. Der NCO-Gehalt der Komponente A) liegt zwischen 30 und 60, bevorzugt 32 und 50 %.

Beispielhaft genannt seien Diisocyanate wie 1,4-Diisocyanatobutan, 1,5-Diisocyanatopentan, 1,6-Diisocyanatohexan, 1,11-Diisocyanatoundecan, Dodecamethylendiisocyanat, 2,2,4- und 2,4,4-Trimethyl-1,6-diisocyanatohexan, 1-Isocyanato-3-isocyanatomethyl-3,5,5-trimethylcyclohexan (Isophorondiisocyanat), 1,3-Diisocyanatocyclobutan, 1,3- und 1,4-Diisocyanatocyclohexan, 4,4'-Bis-(isocyanatocyclohexyl)-methan, 1,2-Bis-(isocyanatomethyl)-cyclobutan, 1,3- und 1,4-Bis-(isocyanatomethyl)-cyclohexan, Hexahydro-2,4- und/oder 2,6-diisocyanatotoluol, Bis-isocyanatomethylnorbornan (Isomerengemisch), 2,5- und 2,6-Bis(isocyanatomethyl)bicyclo[2.2.1]heptan, Lysindiisocyanate, 1-Isocyanato-4(3)-iso-cyanatomethyl-1-methylcyclohexan oder p-Xylylendiisocyanat, 2,3-Bis(8-isocyanatooctyl)-4-octyl-5-hexylcyclohexan bzw. beliebige Mischungen.

Zur Modifizierung können auch Monoisocyanate wie Butylisocyanat, Hexylisocyanat, 2-Ethylhexylisocyanat, Stearylisocyanat und Cyclohexylisocyanat in Mischungen mit Diisocyanaten eingesetzt werden. Desweiteren sind auch höherfunktionelle Isocyanatverbindungen verwendbar, solange die Gesamtfunktionalität der Komponente A) nicht über 2,3 bevorzugt 2,1 liegt. Die aufgeführten Mono- und Diisocyanate können durch Urethan- und/oder Allophanat- und/oder Harnstoff- und/oder Biuret- und/oder Uretdion- und/oder Carbodiimidgruppen modifiziert sein.

Bevorzugt wird als Isocyanatkomponente A) 1,6-Diisocyanatohexan eingesetzt.

Als Isocyanatkomponente B) kommen beliebige Isocyanatverbindungen oder Gemische mit aromatisch gebundenen Isocyanatgruppen mit einem mittleren Molekulargewicht zwischen 148 und 800, vorzugsweise 148 und 250 in Betracht. Die Gesamtfunktionalität der Komponente B) liegt zwischen 1,7 und 2,3, vorzugsweise 1,9 und 2,1 und besonders bevorzugt bei 2.

Beispielhaft genannt seien die isomeren Diisocyanatodiphenylmethane, 2,4- und/oder 2,6-Toluylendiisocyanat, 1,3-Phenylendiisocyanat, 1,4-Phenylendiisocyanat, 4,4'-Biphenylendiisocyanat, 1,5-Naphthylendiisocyanat, 1,4-Naphthylendiisocyanat und 4,4'-Diisocyanatodiphenylether sowie deren Mischungen.

In Abmischungen mit höherfunktionellen und/oder difunktionellen Isocyanatverbindungen können auch Monoisocyanate wie Phenylisocyanat und Toluylisocyanate Verwendung finden.

Auch die Isocyanatkomponente B) kann durch Urethan- und/oder Allophanat- und/oder Harnstoff- und/oder Biuret- und/oder Uretdion- und/oder Carbodiimidgruppen modifiziert sein.

Ganz bevorzugt wird als Isocyanatkomponente B) 2,4- und/oder 2,6-Toluylendiisocyanat eingesetzt.

Die Konzentration an Isocyanatkomponente A) liegt bei 90 bis 10 vorzugsweise 70 bis 20 und besonders bevorzugt zwischen 60 und 30 Teilen auf 100 Teilen Isocyanatkomponente A) und B).

Als Aminosilylverbindungen C) werden Verbindungen mit einer oder mehreren Si-N-Gruppierungen der Formel (I) 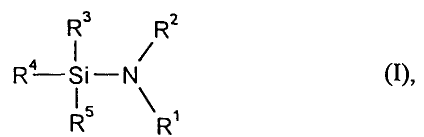 in der
- R¹, R², R³, R⁴ und R⁵: für unabhängig voneinander gesättigte oder ungesättigte, C₁-C₂₂ aliphatische, C₅-C₂₀ cycloaliphatische oder C₆-C₂₄ aromatische, gegebenenfalls mit Cl, Br substituierte Kohlenwasserstoffreste stehen und R¹ auch Wasserstoff bedeuten kann,
eingesetzt.

Bevorzugt bilden R¹ und R² einen Ring mit 2 bis 6 C-Atomen, wobei gegebenenfalls einzelne C-Atome des Ringes durch Heteroatome wie Sauerstoff, Schwefel, Phosphor, oder Stickstoff ersetzt sein können.

Besonders bevorzugt sind Verbindungen wie N-(bis)silyliertes Morpholin, Piperidin und Piparazin.

Bevorzugt handelt es sich bei R² um eine SiR³R⁴R⁵-Gruppierung, beispielsweise Hexamethyldisilazan.

Bei den Resten R³, R⁴ und R⁵ handelt es sich unabhängig voneinander bevorzugt um Methyl-, Ethyl-, Propyl-, Butylgruppen bzw. deren Isomere. Ganz besonders bevorzugt handelt es sich um Methylgruppen.

Erfindungsgemäß einsetzbare Aminosilylverbindungen der Formel (I) sind handelsüblich.

Die Katalysatoren oder Mischungen C) kommen bevorzugt in Konzentrationen von 0,1 bis 10 Teilen bezogen auf 100 Teile Komponente A und B zum Einsatz. Besonders bevorzugt liegt die Konzentration bei 0,8 bis 5 Teilen.

Zur besseren Handhabung können die Katalysatoren gegebenenfalls auch in gelöster From zur Anwendung gelangen.

Die katalytische Trimerisierung wird im Temperaturbereich von ca. 50 bis 140°C, vorzugsweise 80 bis 125°C durchgeführt.

Nach Beendigung der Trimerisationsreaktion wird der Katalysator bevorzugt durch Zusatz eines Katalysatorgiftes desaktiviert. Als geeignete Verbindungen sind hier bevorzugt monofunktionelle bzw. polyfunktionelle Alkohole bzw. Mercaptane und besonders bevorzugt Carbonsäuren zu nennen.

Nach der Trimerisationsreaktion und gegebenenfalls Abstoppung mit Desaktivatoren bzw. Katalysatorgiften werden aus dem Reaktionsgemisch überschüssige monomere destillierbare Komponenten A bzw. B vorzugsweise durch Dünnschichtdestillation im Vakuum enfernt. Der Restgehalt an monomeren destillierbaren Komponenten A und B zusammen liegt unter 0,7 %, vorzugsweise unter 0,5 % und besonders bevorzugt unter 0,1 %.

Den erfindungsgemäßen Verfahrensprodukten können anschließend Lösemittel zugesetzt werden. Der Festkörpergehalt der hergestellten Produkts beträgt dann mindestens 40 Gew.-% bevorzugt mindestens 60 Gew.-%.

Geeignete Lösungsmittel hierfür sind beispielsweise Toluol, Xylol, Cyclohexan, Chlorbenzol, Butylacetat, Ethylacetat, Ethylglykolacetat, Pentylacetat, Hexylacetat, Methoxypropylacetat, Tetrahydrofuran, Dioxan, Aceton, Methylethylketon, Testbenzin, höher substituierte handelsübliche Aromaten wie Solvent Naphtha®, Solvesso®, Shellsol®, Isopar®, Nappar® und Diasol®, Schwerbenzol, Tetralin, Dekalin und Alkane mit mehr als 6 Kohlenstoffatomen sowie Gemische derartiger Lösungsmittel.

Bei den durch das erfindungsgemäße Verfahren hergestellten Polyisocyanaten handelt es sich um weitgehend farblose, lagerstabile, unter dem Einfluß von Luftfeuchtigkeit auch alleine aushärtbare Beschichtungsmaterialien.

Bevorzugt werden sie als Vernetzer in 2-Komponentensystemen (2 K) mit an sich bekannten isocyanatreaktiven Verbindungen eingesetzt. Hierzu zählen beispielsweise hydroxyfunktionelle Polyether, -ester, -amide, -carbonate, -acrylate, -butadiene bzw. Mischtypen der genannten hydroxyfunktionellen Polymeren, niedermolekulare Di- und Polyole, Di- und Trimerfettalkohole sowie aminofunktionelle Verbindungen.

Mit blockierten isocyanatreaktiven Verbindungen können auch Einkomponentensysteme formuliert werden. Weiterhin können die nach dem erfindungsgemäßen Verfahren hergestellten Produkte auch in blockierter Form als oder in Beschichtungsmaterialien eingesetzt werden. Die Trocknung erfolgt bei höheren Temperaturen bis ca. 200°C.

Neben den erfindungsgemäßen Verfahrensprodukten können in den Beschichtungen auch andere Hilfs- und Zusatzmittel wie beispielsweise die üblichen Katalysatoren, Benetzungsmittel, Verlaufsmittel, Hautverhinderungsmittel, Antischaummittel, Lösungsmittel, Mattierungsmittel wie beispielsweise Kieselsäure, Aluminiumsilikate und hochsiedende Wachse, viskositätsregulierende Stoffe, Pigmente, Farbstoffe, UV-Absorber, Stabilisatoren gegen thermischen bzw. oxidativen Abbau eingesetzt werden.

Die erhaltenen Beschichtungsmaterialien können zur Beschichtung beliebiger Substrate wie beispielsweise Holz, Kunststoffe, Leder, Papier, Textilien, Glas, Keramik, Putz, Mauerwerk, Metalle oder Beton verwendet werden. Sie lassen sich mit üblichen Applikationsmethoden wie Spritzen, Streichen, Fluten, Gießen, Tauchen, Walzen aufbringen. Die Beschichtungsmittel können sowohl in Form von Klarlacken als auch in Form pigmentierter Lacke verwendet werden.

Die aus den erfindungsgemäßen Produkten hergestellten Beschichtungen härten bei Raumtemperatur (ca. 20°C) innerhalb von einigen Minuten bis Stunden zu hochwertigen harten Überzügen aus. Die Härtung kann jedoch auch bei tieferen Temperaturen (bis -5°C) oder beschleunigt bei höheren Temperaturen (80 bis 200°C) erfolgen.

### Beispiele

Alle Angaben bezüglich "Teilen" und "%" beziehen sich auf das Gewicht.

### Beispiel 1

In einer Rührapparatur, bestehend aus Planschliffkolben, Innenthermometer, Metallrührer, Tropftrichter und Rückflußkühler mit Trockenrohraufsatz, werden 608 g (3,5 mol) 2,4-Toluylendiisocyanat und 391 g (2,3 mol) 1,6-Hexamethylendiisocyanat vorgelegt und unter Stickstoffatmosphäre auf 110°C aufgeheizt. Nachfolgend tropft man 15 g (1,5 %) Hexamethyldisilazan zu dieser Mischung und rührt so lange bei einer Temperatur von 115°C, bis ein NCO-Gehalt von 39 % erreicht ist (ca. 8 Stunden). Die Reaktionsmischung wird abgekühlt und im Anschluß durch Dünnschichtdestillation (Temperatur 180°C, 0,5 mbar) von überschüssigem Monomer befreit. Das erhaltene Festharz weist 60 %ig gelöst in Butylacetat folgende Kenndaten auf:

| | |
|---|---|
| titrierter NCO-Gehalt | 11,4 % |
| Festkörpergehalt | 60 % |
| Viskosität | ca. 500 mPas |
| fr. HDI | 0,15% |
| fr. TDI | 0,18% |
| molares Einbauverhältnis TDI/HDI | 1,2 |

### Beispiel 2

Man verfährt wie in Beispiel 1, jedoch werden 15 g N-Trimethylsilylmorpholin als Katalysator eingesetzt. Nach 6 Stunden ist ein NCO-Gehalt von 38,5 % erreicht wobei die Reaktion mit 10,4 g Butanol bei 50°C abgebrochen wird. Nach Abtrennung überschüssiger Monomerer und Lösen in Butylacetat wird ein fast farbloses Produkt mit folgenden Kenndaten erhalten:

| | |
|---|---|
| titrierter NCO-Gehalt | 11,0 % |
| Festkörpergehalt | 60 % |
| Viskosität | ca. 600 mPas |
| fr. HDI | 0,1 % |
| fr. TDI | 0,2 % |

### 3. Vergleichsbeispiel (nicht erfindungsgemäß)

Man verfährt wie unter Beispiel 1, jedoch werden als Katalysator 1,3 g einer Mannichbase auf Basis Phenol/Dimethylamin, 40 %ig in Butylacetat eingesetzt. Bei einem NCO-Gehalt von 38 % wird die Reaktion durch Zugabe von 0,3 g Benzoylchlorid abgestoppt. Die direkte Analyse des Gehaltes an freien Monomeren ergibt ein fast ausschließlicher Einbau von TDI in das Isocyanurat. Der freie HDI-Gehalt bleibt gegenüber dem Anteil in der Ausgangsmischung fast unverändert.

### 4. Vergleichsbeispiel (nicht erfindungsgemäß)

Man verfährt wie in Beispiel 1, jedoch werden 20 g (2 % auf Gesamtansatz) N,N'-Bis-(Trimethylsilyl)-N,N'-dibutylharnstoff als Katalysator eingesetzt. Selbst nach 12 Stunden Reaktion ist der NCO-Gehalt der Lösung nur schwach auf 45,1 % abgesunken. Zielwert (siehe Beispiel 1): ca. 38 %. Dieser Katalysatortyp vermag somit nicht eine Mischtrimerisation von Hexamethylendiisocyanat und Toluylendiisocyanat auszuführen.

## Patentansprüche

1. Verfahren zur Herstellung von Isocyanuratgruppen enthaltenden Polyisocyanaten dadurch gekennzeichnet, daß
10 bis 90 Teile einer niedermolekularen Isocyanatkomponente A) mit aliphatisch gebundenen Isocyanatgruppen, eines mittleren Molekulargewichts von 128 bis 800 und einer mittleren NCO-Funktionalität von 1,7 bis 2,2
und
90 bis 10 Teile einer niedermolekularen Isocyanatkomponente B) mit aromatisch gebundenen Isocyanatgruppen, eines mittleren Molekulargewichts von 148 bis 800 und einer mittleren NCO-Funktionalität von 1,7 bis 2,2 unter katalytischer Trimerisation
mit
0,1 bis 10 Teilen einer Aminosilylverbindung C) pro 100 Teile Isocyanatkomponente A) und B)
zur Reaktion gebracht werden und im Anschluß überschüssige monomere destillierbare Isocyanatkomponente A) und B) bis auf einen Gehalt von zusammen unter 0,7 Gew.-% entfernt werden.

2. Verfahren nach Anspruch 1 dadurch gekennzeichnet, daß als Isocyanatkomponente A) und B) Diisocyanate eingesetzt werden.

3. Verfahren nach Anspruch 1, dadurch gekennzeichnet, daß 20 bis 70 Teile einer niedermolekularen Isocyanatkomponente A) und 80 bis 30 Teile Isocyanatkomponente B) eingesetzt werden.

4. Verfahren nach Anspruch 1, dadurch gekennzeichnet, daß als Isocyanatkomponente A) 1,6-Diisocyanatohexan und als Isocyanatkomponente B) 2,4- und/oder 2,6-Toluylendiisocyanat eingesetzt werden.

5. Verfahren nach Anspruch 1, dadurch gekennzeichnet, daß 0,8 bis 5 Teile einer Aminosilylverbindung C) pro 100 Teile Isocyanatkomponente A) und B) als Katalysator eingesetzt werden.

6. Verfahren nach Anspruch 1, dadurch gekennzeichnet, daß als Katalysator C) N-silylierte Heterocyclen eingesetzt werden.

7. Verfahren nach Anspruch 1, dadurch gekennzeichnet, daß als Katalysator C) N-silylierte cyclische Amine, die noch mindestens ein weiteres Heteroatom im Ring enthalten, eingesetzt werden.

8. Nach Anspruch 1 hergestellte Produkte.

9. Verwendung der nach Anspruch 1 hergestellten Produkte als bzw. in Beschichtungsmaterialien.

## Claims

1. Process for the production of polyisocyanates containing isocyanurate groups, characterised in that
10 to 90 parts of a low molecular weight isocyanate component A) having aliphatically attached isocyanate groups, an average molecular weight of 128 to 800 and an average NCO functionality of 1.7 to 2.2
and
90 to 10 parts of a low molecular weight isocyanate component B) having aromatically attached isocyanate groups, an average molecular weight of 148 to 800 and an average NCO functionality of 1.7 to 2.2 are reacted in a catalytic trimerisation reaction
with
0.1 to 10 parts of an aminosilyl compound C) per 100 parts of isocyanate component A) and B)
and excess, distillable isocyanate components A) and B) are subsequently removed down to a combined content of below 0.7%.

2. Process according to claim 1, characterised in that diisocyanates are used as the isocyanate components A) and B).

3. Process according to claim 1, characterised in that 20 to 70 parts of a low molecular weight isocyanate component A) and 80 to 30 parts of isocyanate component B) are used.

4. Process according to claim 1, characterised in that 1,6-diisocyanatohexane is used as isocyanate component A) and 2,4- and/or 2,6-tolylene diisocyanate as isocyanate component B).

5. Process according to claim 1, characterised in that 0.8 to 5 parts of an aminosilyl compound C) are used as catalyst per 100 parts of isocyanate components A) and B).

6. Process according to claim 1, characterised in that N-silylated heterocyclics are used as the catalyst C).

7. Process according to claim 1, characterised in that N-silylated cyclic amines still containing at least one further ring heteroatom are used as the catalyst C).

8. Products produced according to claim 1.

9. Use of the products produced according to claim 1 as or in coating materials.

## Revendications

1. Procédé de préparation de polyisocyanates contenant des radicaux isocyanurate, caractérisé en ce que l'on fait réagir
10 à 90 parties d'un composant isocyanate A) de faible poids moléculaire, avec des radicaux isocyanate liés de manière aliphatique, ayant un poids moléculaire moyen allant de 128 à 800 et une fonctionnalité NCO moyenne allant de 1,7 à 2,2, et
90 à 10 parties d'un composant isocyanate B) de faible poids moléculaire, avec des radicaux isocyanate liés de manière aromatique, ayant un poids moléculaire moyen allant de 148 à 800 et une fonctionnalité NCO moyenne allant de 1,7 à 2,2, par trimérisation catalytique avec
0,1 à 10 parties d'un composé aminosilylé C), par 100 parties des composants isocyanate A) et B),
et, en outre, on sépare les composants isocyanate A) et B) monomères en excès, distillables, jusqu'à une teneur globale inférieure à 0,7%.

2. Procédé suivant la revendication 1, caractérisé en ce que l'on met en oeuvre comme composants isocyanate A) et B), des diisocyanates.

3. Procédé suivant la revendication 1, caractérisé en ce que l'on met en oeuvre 20 à 70 parties d'un composant isocyanate A) de faible poids moléculaire et 80 à 30 parties de composant isocyanate B).

4. Procédé suivant la revendication 1, caractérisé en ce que l'on met en oeuvre comme composant isocyanate A), le 1,6-diisocyanatohexane et comme composant isocyanate B), le 2,4- et/ou 2,6-toluylènediisocyanate.

5. Procédé suivant la revendication 1, caractérisé en ce que l'on met en oeuvre 0,8 à 5 parties d'un composé aminosilylé, par 100 parties de composants isocyanate A) et B), comme catalyseur.

6. Procédé suivant la revendication 1, caractérisé en ce que l'on met en oeuvre comme catalyseur C), des hétérocycles N-silylés.

7. Procédé suivant la revendication 1, caractérisé en ce que l'on met en oeuvre comme catalyseur C), une amine cyclique N-silylée, qui contient encore au moins un autre hétéroatome dans le cycle.

8. Produits préparés suivant la revendication 1.

9. Utilisation des produits préparés suivant la revendication 1, comme ou dans des matériaux de revêtement.
